# EUROPEAN PATENT SPECIFICATION

(11) **EP 4 605 729 B1**
(45) Date of publication and mention of the grant of the patent: **23.09.2026**
(21) Application number: 23786107.5
(22) Date of filing: 11.10.2023
(51) Int. Cl.: G01N 21/552, A61B 5/1459, G01N 21/31, G01N 21/359

(54) **MULTILAYER PROTECTIVE STRUCTURE IN AN IMPLANTABLE OPTICAL SENSOR**
MEHRSCHICHTIGE SCHUTZSTRUKTUR IN EINEM IMPLANTIERBAREN OPTISCHEN SENSOR
STRUCTURE DE PROTECTION MULTICOUCHE DANS UN CAPTEUR OPTIQUE IMPLANTABLE

(30) Priority: 18.10.2022 EP 22202320
(43) Date of publication of application: 27.08.2025
(73) Proprietor: Indigo Diabetes N.V., 9052 Zwijnaarde (BE)
(72) Inventor: ORDONEZ ORELLANA, Juan Sebastian, 9000 Gent (BE); DELBEKE, Danaë, 9050 Gentbrugge (BE)
(74) Representative: Ipsilon Belgium
(86) International application number: PCT/EP2023/078194
(87) International publication number: WO 2024/083609

(56) References cited:
- WO-A1-2014/006540
- WO-A1-2018/185033
- KR-B1- 101 052 504
- US-A- 5 770 462
- US-A- 5 991 488
- US-A1- 2002 076 154
- US-B2- 7 863 052

## Description

### Field of the invention

The present invention relates, in general, to an implantable optical sensor. The present invention further relates, in general, to a method of manufacturing an implantable optical sensor. The present invention further relates, in general, to a use of a multilayer protective structure for protecting an implantable optical sensor.

### Background art

Implantable biosensors are an important class of biosensors because of their ability to provide continuous data on the levels of a target substance (e.g., an analyte) .This enables trends and changes in analyte levels over time to be monitored without any need for intervention from either the patient or clinician. As such, implantable biosensors have great potential in the diagnosis, monitoring, management and treatment of a variety of disease conditions.

Examples of next-generation sensors are implantable glucose bio-sensors. To date, implantable glucose sensors are typically based on surface chemical reactions. Such sensors are very stable, accurate and sensitive in vitro. However, once the sensors are implanted, the stability and reliability reduces dramatically after a few days owing in large part to fouling of the sensor surface by proteinaceous material. They can therefore not be used for long term implantation.

Alternative sensors under study are based on spectrometric devices (optical sensors) which should allow long term in vivo operation thus justifying the surgical procedure to implant the device. However, such sensors suffer from corrosion or deterioration when exposed to bodily fluids, limiting their longevity. Optical sensors therefore have a need for protective layers or coatings, in particular of the optical interaction area, where the optical waveguide is exposed to bodily fluids in order to measure an analyte.

Document WO 2014/006540 A1 describes a device comprising a nano-structure, wherein said nano-structure is made of electrically conductive material and wherein said nano- structure is covered by a barrier coating comprising Ti, Zr, Hf, Nb, Ta, Mo, Sc, Y, Ge, La, Ce, Pr, Nd, Sm, Eu, Gd, Dy, Ho, Er, Tm, Vb, Lu, Sr, Al, B, Ba, Bi, and/or Mg oxide in a thickness of at least about 1 nm, wherein said barrier coating is deposited by atomic layer deposition.

Document US 2012/0226118 A1 describes a sensor for sensing a substance such as for example glucose equipped with a semi-permeable element, e.g. a membrane and/or layer to prevent adhesion of particles, such as proteins, on the sensing surface while allowing the particles to be characterised to pass.

Document US 2011/0090484 A1 describes an optical sensor unit for evanescence wave spectroscopy using an infrared light waveguide made of nanocrystalline diamond (NCD) or coated with a thin layer of NCD on polycrystalline diamond.

Document WO 2018/185033 A1 describes optical assembly comprising a substrate and at least one optical microstructure integrated with the substrate. The optical microstructure is positioned to form an optical interaction area on a part of a surface of the substrate.

Despite the huge efforts that have been made in this area, the development of implantable biosensors still presents major challenges, such as the foreign-body response, the biosensor's response and stability, continuous monitoring, power supply, and data transmission.

In order to overcome these limitations, specific requirements must be met for implantable biosensors, such as the use of more flexible and biocompatible biomaterials (biodegradability and/or bioresorbability may also be required in some contexts), miniaturization, and reliability. Thus, the following design parameters are crucial in the development of implantable biosensors.

The present inventors have found that known materials used as a protective layer for optical sensors such as silicon carbide (referred to hereinafter as "SiC"), carbon-rich silicon (referred to hereinafter as "c-ri Si" or "CRAS") or Silicon oxides (e.g., thermally grown Silicon dioxide) suffer from several drawbacks, for example they require a large thickness in order to provide sufficient protection (e.g., >300 nm), thereby compromising the miniature size (i.e., increasing the size) of the optical sensor, as well as reducing the optical performance (i.e., increasing optical losses) of the optical sensor.

There is a need in the art to provide an implantable sensor with improved longevity after implantation, improved protection of the optical sensing area and/or improved sensitivity, for stable and reliable sensing of substances.

### Summary of the invention

The present inventors have developed an implantable optical sensor which is particularly suited for applications involving direct and prolonged contact with a (bodily) fluid (e.g., chemical or biological substances) which over time adversely affects the optical properties and the lifetime of the implantable optical sensor. Surprisingly, it was found that specific combinations of certain protective layers enable sufficient protection of the implantable optical sensor at much lower thicknesses (e.g. <60 nm) compared to known materials used as protection layers on optical sensors. Moreover, as will be shown in the appended examples, the inventors have further found that protection layers of the present invention exhibit significantly improved optical performance in specific wavelength domains of crucial interest for measuring an analyte such as glucose. This allows more accurate, sensitive, stable and reliable sensing compared to known optical sensors.

Hence, according to a first aspect of the present invention, there is provided an implantable optical sensor comprising: at least one optical microstructure configured for evanescent field sensing, the at least one optical microstructure being positioned to form an optical interaction area on a part of a surface of the implantable optical sensor, wherein the implantable optical sensor further comprises a multilayer protective structure covering at least the optical interaction area, the multilayer protective structure comprising a first layer deposited on a second layer, the first layer being a hafnia layer and the second layer being an alumina layer.

Advantageously, the multilayer protective structure of the present invention ensures a combination of improved stability and reduced optical loss compared to a single layer of typical materials used, especially when the single layer is of similar or same thickness to the multilayer protective structure. Examples of a single layer are Silicon Carbide (SiC), a carbon-rich amorphous silicon layer (e.g., c-ri Si).

Furthermore, at the optical interaction area, an optical signal (e.g., light or radiation) can be confined within the optical microstructure, wherein only a small portion of the optical signal being the evanescent field may extend out into an external medium (e.g. the substrate material and/or the optical interaction area). This evanescent field falls off exponentially as the distance from the optical microstructure surface increases. The evanescent field is used to interact with the environment for e.g. optical trapping, sensing, exciting, etc. In one of the embodiments of the invention of the present invention, the optical microstructure may have the protective layer as its upper cladding The extending evanescent field, preferably the evanescent tail in the optical interaction area may be used for sensing purposes.

According to a second aspect of the present invention, there is provided a method of manufacturing an implantable optical sensor, the method comprising: providing a substrate; and providing with the substrate at least one optical microstructure for evanescent field sensing, the at least one optical microstructure being positioned to form an optical interaction area on a part of a surface of the substrate, wherein the method further comprises providing a multilayer protective structure covering at least the optical interaction area, the multilayer protective structure comprising a hafnia layer deposited on an alumina layer.

According to a third aspect of the present invention, there is provided a use of a multilayer protective structure for protecting an implantable optical sensor, the multilayer protective structure covering at least an optical interaction area in the implantable optical sensor, the multilayer protective structure comprising a hafnia layer deposited on an alumina layer.

Additional and alternative objects of the present invention may be understood from the following.

### Brief description of the drawings

The present invention will be discussed in more detail below, with reference to the attached drawings, in which
Fig. 1 shows a schematic two dimensional cross sectional view of an implantable optical sensor according to a first embodiment of the present invention;
Fig. 2 shows a schematic two dimensional cross sectional view of an implantable optical sensor according to a second embodiment of the present invention;
Fig. 3 shows a schematic two dimensional cross sectional view of an implantable optical sensor according to a third embodiment of the present invention;
Fig. 4 shows a schematic two dimensional cross sectional view of an implantable optical sensor according to a further embodiment of the present invention;
Fig. 5 shows a schematic representation in cross sectional view of an even further embodiment of the implantable optical sensor of the present invention;
Fig. 6 shows a schematic representation in cross sectional view of an even further embodiment of the implantable optical sensor of the present invention;
Fig. 7 shows a schematic representation of comparative results of an experiment performed on an implantable optical sensor according to an embodiment of the present invention;
Figs. 8A-8C show graphical representations of an experiment performed on an implantable optical sensor comprising a hafnia-alumina-hafnia protective multilayer according to an embodiment of the present invention;
Figs. 9A-9C show graphical representations of an experiment performed on an implantable optical sensor comprising a carbon-rich silicon protective layer;
Figs. 10A-10C show graphical representations of an experiment performed on an implantable optical sensor comprising a hafnia-alumina-hafnia protective multilayer deposited on a carbon-rich silicon protective layer;
Figs. 11A-11C show schematic two dimensional cross sectional views of an optical interaction area of an implantable optical sensor according to several embodiments of the present invention.

### Description of embodiments of the invention

The present invention will be described with respect to particular embodiments of the invention and with reference to certain drawings but the invention is not limited thereto but only by the claims.

Furthermore, the various embodiments of the invention, although referred to as "preferred" are to be construed as exemplary manners in which the invention may be implemented rather than as limiting the scope of the invention.

The term "comprising", used in the claims, should not be interpreted as being restricted to the elements or steps listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a device comprising A and B" should not be limited to devices consisting only of components A and B, rather with respect to the present invention, the only enumerated components of the device are A and B, and further the claim should be interpreted as including equivalents of those components.

The term "hafnia" as used herein refers to a material containing hafnium compound, such as hafnium oxide (HfO₂), as well as doped and oxygen-deficient hafnium oxide.

The term "alumina" refers to a material containing aluminium compound, such as aluminium oxide (Al₂O₃), as well as doped and oxygen-deficient aluminium oxide

The present invention in general relates to the use of optical microstructures for many applications including an optical sensor and an implantable optical sensor which require direct and/or long term interaction of a surface of the optical microstructure with a hostile environment. These optical microstructures are configured to guide radiation e.g. via waveguide structures, for which the evanescent tail of the guided radiation is interacting with the environment.

Corrosion is a chemical-physical process in which a material in an initial chemical composition undergoes a chemical reaction with a secondary element from an adjacent material or the surrounding environment (solid, liquid, gaseous or combination of all) thus reducing its enthalpy or thermodynamically available energy (Gibb's free energy) under given ambient conditions (pressure, volume, temperature). The resulting compound is mostly less chemically active than the original material state (chemically inert to the given environmental conditions). If the compound formation is continuous, corrosion will eventually degrade the initial properties of the initial material. The driving force can be initiated either from the material or from its environment or from the combination. From an energy point of view, a system (e.g. a material or material composition) strives towards a minimal free energy, undergoing reactions with its environment towards a product that exhibits a lower free energy than the educts in the system.

The reaction product during a corrosion reaction is of different chemical but also of different physical properties compared to its individual educts. For example, a metal that undergoes oxidation reduces its energy to a less chemically active metal-oxide. This involves a change in its underlying quantum structure, leading to a change in band gap and to its properties such as conduction, thermal and chemical behaviour. The reaction has also an impact on the crystallographic structural configuration of the material, leading to a change in its volume upon the chemical reaction. This reaction induces mechanical forces which are exerted on the new material and on adjacent layers, e.g. the initial surface. If the volume of the corrosion product is much larger than the surface area of the educts undergoing the reaction, the stress will accumulate until it induces cracks in the system, releasing the inbuilt stress and so reducing the stored energy. Examples of undergoing a chemical-physical change in order to reduce its energy are flaking of an oxidized material, pitting corrosion, stress crack corrosion, contact corrosion, etc.

A hostile environment, in the sense of the above definition of corrosion, is one that in a liquid, solid or gaseous form contains chemical elements in a state with energy that is capable of inducing corrosion on a material exposed to this environment. Pure water, as an example has a high thermodynamic activity which is reduced through the incorporation of solutes into the liquid matrix. Water can thus be considered a hostile or corrosive environment for materials such as Si, silicon oxide (SiOx) or silicon nitride. Bodily fluids, containing dissolved oxygen, hydrogen peroxide, chlorine, and multiple other highly oxidizing and other thermodynamically active agents represent an environment that subjects multiple materials to high risk of corrosion.

Hostile environments include, but are not limited to exposure to body tissues and fluids (such as blood or interstitial fluid), in-vitro and in-vivo, vegetal tissues and fluids, fluids in chemical processes such as fermentation tanks and bio reactors, aquacultures including sea, lakes, rivers and aqua tanks and petrochemical fluids, water and corrosive gases. The interaction with a hostile environment will degrade the optical, mechanical and electrical performance of the exposed surface of the optical microstructure, e.g. by corrosion or dissolution. As an example, optical grade silicon (Si) and silicon nitride structures used to transport light through photonic microsystems dissolve, pit and disintegrate when used in aqueous or aggressive chemical environments. In applications where the light from the photonic microsystems is intended to interact with the environment, the deterioration of the surface's quality has a direct impact on the performance of the optical assembly. It is also known that optical microstructures configured for evanescently contacting the environment are heavily impacted by the surface conditions of the optical microstructures.

Therefore a protective structure is added in the present invention embodiment, as described in more detail below, which protects the interaction area which has direct interaction with the environment. This coating does not deteriorate the performance of the optical microstructures, nor modifies the functionality of the optical microstructure, as it could be expected by persons skilled in the art. Specifically for an optical sensor it is desired that this protective structure does not adversely affect the desired optical functionalities of the system.

A corrosion resistant material as used in this description refers to a material, which under specific physical environmental conditions (temperature, volume, pressure) either has a state of minimum energy that cannot be further minimized by undergoing a detrimental reaction (oxidation) with the surrounding environment (e.g. solid, gas or liquid); or exhibits a strong intramaterial bond, that the thermodynamic activity or chemical potential of the environment cannot disrupt, unless further energy is added into the system. The strength of the system can be given by the level of crystallinity (reduced energy) and its strong intermaterial bonds (present in for example Diamond, Diamond-like carbon (DLC)), the reduced energy and strong bond through preceded by oxidation (e.g. Aluminium oxide (Al₂O₃), Titanium oxide) or the combination of both (e.g. Sapphire, pure crystal Al₂O₃).

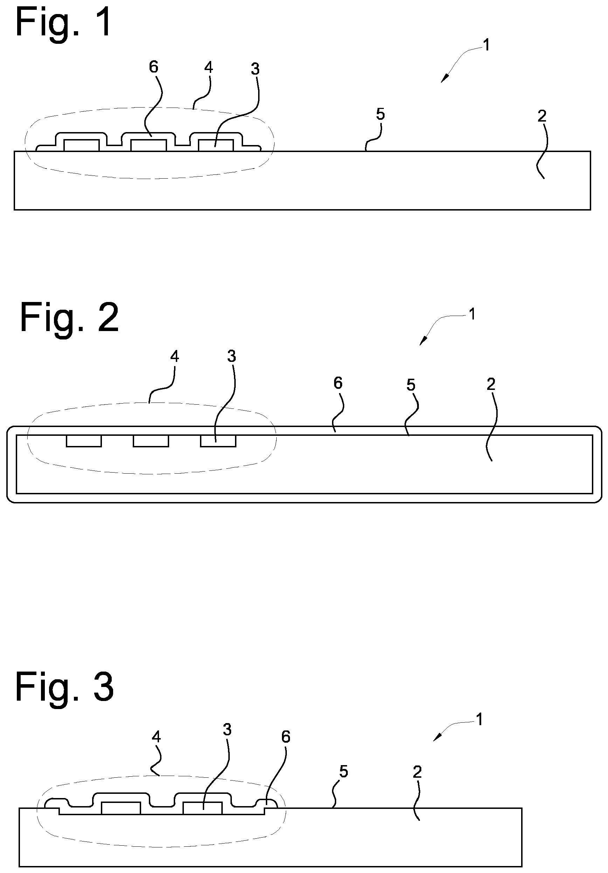

Fig. 1 shows a schematic two dimensional cross sectional view of a first embodiment of an implantable optical sensor 1 in accordance with the present invention. The implantable optical sensor 1 comprises an optical microstructure 3 deposited on the optical sensor 1. The optical sensor 1 may comprise a substrate 2. The substrate 2 may be Silicon (Si) or SOI based. The optical microstructure 3 is configured for evanescent field sensing and is positioned to form an optical interaction area 4 on a part of a surface 5 of the implantable optical sensor 1 or of the substrate 2. In the embodiment shown in Fig. 1, the optical microstructure 3 is integrated with the substrate 2 by being provided on top of the substrate 2. The implantable optical sensor 1 further comprises a multilayer protective structure 6 covering at least the optical interaction area 4. In the embodiment shown in Fig. 1, the implantable optical sensor 1 comprises a plurality of optical microstructures 3 provided on top of the optical sensor 1 or the substrate 2 and covered by the (thin) protective structure 6. As a result, evanescent waves from the at least one optical microstructure 3 can still optically interact with the environment in the optical interaction area 4.

The present invention embodiments of the invention, such as the one shown in Fig. 1, allows to obtain a surface protection using the protective structure 6 that maintains the capability of optical interaction between the optical microstructure 3 and the external environment of the optical assembly 1. The protective structure 6, of which exemplary embodiments of the invention are discussed below, enable optical interaction of the optical microstructure and the environment without changing the desired optical functionalities over time. Fabrication of the optical sensor 1 can be executed using various techniques, such as electron beam technology, photolithographic process, CMOS technology or using silicon based technology, or a combination thereof. This can include material etching processes (e.g. wet etching, dry etching) and other typical back-end-of-line processes (e.g. metallization) or steps involving a heterogeneous integration of other micro-components on the substrate (e.g. flip-chipping, bonding) which are as such known to the person skilled in the art.

Fig. 2 shows a schematic two dimensional cross sectional view of an optical sensor 1 according to a second embodiment of the present invention. In this embodiment, the optical microstructure 3 is embedded in or integrated within the substrate 2, and the optical interaction area 4 as a result is planar and level with the surface 5. The substrate 2 may be etched to allow the depositing of the optical microstructure 3 in the etched regions. The etched regions may form valleys having similar dimensions as the optical microstructure 3, the result of which would allow the optical microstructure 3 to be integrated within the substrate 2.

Furthermore, the protective structure 6 in this embodiment is present on the entire (top) surface 5 of the optical sensor 1 or the substrate 2, and even extends onto a back side of the optical sensor 1 or the substrate 2. This may be advantageous in view of the processing steps for applying the protective structure 6. Also variations of the embodiments of the invention shown in Fig. 1 and Fig. 2 are possible, e.g. with the protective structure 6 present over the entire surface 5 (including front side, back side and edges in between the front side and back side. It is noted that for a proper operation of the optical sensor 1 in a hostile environment, presence of the protective structure 6 over the optical interaction area 4 is sufficient. The remaining part of the optical assembly 1 is then not necessarily covered with the protective structure 6.

In a further embodiment of the present invention the protective structure 6 is conformal to an external surface associated with the optical interaction area 4. A conformal layer implementation of the protective structure 6 covers all surfaces with a coating of a substantially uniform thickness which means that the thickness of the conformal protective structure 6 will be substantially same over the entire optical interaction area 4 where the protective structure 6 is present.

Fig. 3 shows a schematic two dimensional cross sectional view of an optical assembly 1 according to a further embodiment of the present invention. In this embodiment, the optical microstructure 3 is integrated with the substrate 2 by being etched in the substrate 2 and the optical interaction area 4 as a result is a patterned valley in the surface 5. The protective structure 6 in this embodiment is conformal to the etched valleys and to the surface 5 of the optical microstructure 3.

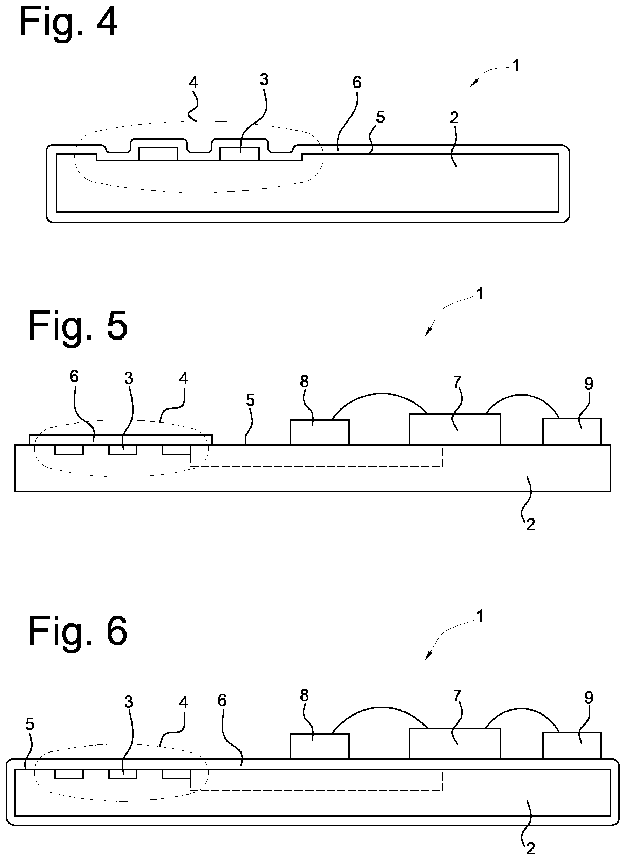

In an even further embodiment, the protective structure 6 extends onto the back and edge sides of the substrate 2. This may be advantageous in view of the processing steps for applying the protective structure 6. Fig. 4 shows a schematic two dimensional cross sectional view of an optical assembly 1 according to this embodiment of the present invention. Similar to the embodiment shown in Fig. 3, here the optical microstructure 3 is etched in the substrate 2 and the optical interaction area 4 as a result is a patterned valley in the surface 5.

### Protective structure

The term "protective structure" used herein refers to the term "multilayer protective structure", said terms will be used interchangeably herein.

As is understood, the protective structure 6 described hereon correspond in many aspects and features with the protective structure 6 described with reference to Figs. 1 to 4. Although not shown in Figs. 1 to 4, the protective structure 6 is a multilayer protective structure 6.

In embodiments of the invention, each layer of the multilayer protective structure may deposited using atomic layer deposition (ALD). For example, thermal ALD or plasma-enhanced ALD. Alternatively, each layer of the multilayer protective structure may be deposited by sputtering, pulsed laser deposition (PLD), a molecular beam epitaxy (MBE) deposition technique, chemical vapour deposition (CVD) such as plasma enhanced (PLCVD), low pressure (LPCVD) or aerosol (ASCVD), or a combination thereof such as atomic layer chemical vapour deposition (ALCVD). For example, a deposition method for conformal coating is ALD (e.g. Thermal ALD or plasma-enhanced ALD). As will be understood, "a layer" such as a hafnia layer and/or an alumina layer can be created by multiple rounds of any of the aforementioned methods of deposition, and still qualifies as a layer for the purpose of the present invention.

The external surface associated with the optical interaction area 4 may span across the optical microstructure 3 which is e.g. patterned or embossed in the surface 5. For a patterned optical interaction area 4, the conformal protective structure 6 may cover the steps, corners and curves of the optical microstructure 3 in a substantially uniform manner (as shown in the embodiment of Fig. 1).

In embodiments of the invention, the multilayer protective structure 6 may comprise one or more materials with corrosion-protection characteristics. Thus, the multilayer protective structure 6 would be considered as a corrosion resistant structure, and preferably optically transparent. Thus, the multilayer protective structure provides further improved mechanical properties (e.g., protection against corrosion of any part or parts of the implantable optical structure), while allowing for optical application in harsh environments (i.e., for evanescent field sensing). With reference to the embodiments of the invention shown in Figs. 1 to 4, the protective structure 6 protects the optical microstructure 3 (and surrounding parts of the surface 5 in the optical interaction area 4) against deterioration (corrosion and dissolution) when exposed to a corrosive hostile environment.

To allow evanescent field sensing, the protective structure 6 is selected in such a manner that it does not adversely affect the optical properties of the optical microstructure 3. In the present invention, the protective structure 6 comprises an alumina layer and a hafnia layer deposited on the alumina layer.

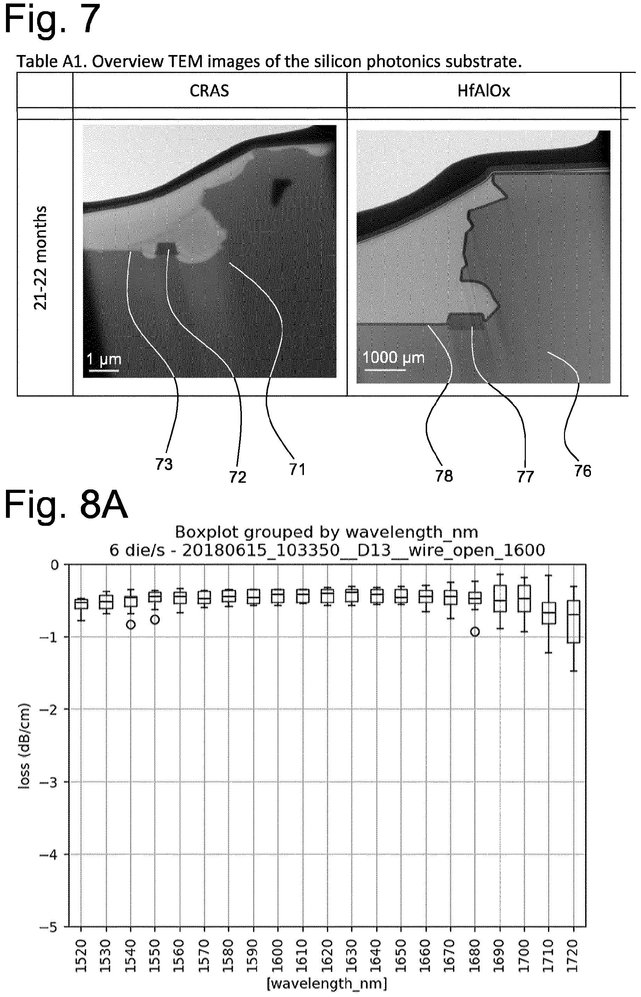

Typical materials used as a protective layer are from the group of carbon-rich amorphous silicon (CRAS), Silicon Carbide (SiC), Diamond Like Carbon (DLC), TiO₂, or Al₂O₃. These materials are known for being poor optical waveguides, therefore a thin coating is needed to ensure that said protective layer does not adversely change the light propagation in the underlying waveguide of the optical microstructure 3. However, by providing a thin coating the protective characteristics of said protective layer would diminish. The multilayer protective structure 6 according to the present invention overcomes these drawbacks, and will be explained in further detail in the experimental section below and with reference to Figs. 7, 8A to 8C, 9A to 9C and 10A to 10C. It has been shown that the multilayer protective structure 6 may have an optical loss less than 3 dB/cm over most of the IR ranges, preferably less than 2 dB/cm, particularly over most of the near IR wavelength range outperforming the typical materials used as a protective layer. Again, more details will be provided in the experimental section below and with reference to Figs. 7, 8A to 8C, 9A to 9C and 10A to 10C.

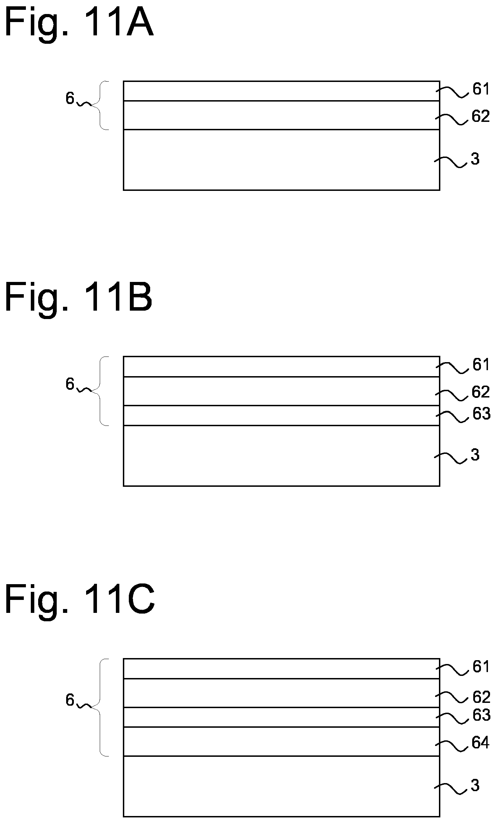

Figs. 11A-11C shows schematic two dimensional cross sectional views of an optical interaction area of an implantable optical sensor according to several embodiments of the present invention. As can be understood, Figs. 11A-11C each show a cut-out of the cross-section of the optical microstructure 3 and the multilayer protective structure 6 deposited thereon. Therefore, the multilayer protective structure 6 in each of Figs. 11A-11C covers the optical microstructure 3, preferably at least the optical interaction area 4 formed by the optical microstructure 3, as described herein with reference to Figs. 1-6.

Note that Figs. 11A-11C are not illustrated to scale, therefore the dimensions of the optical microstructure 3 and the layers of the multilayer protective structure 6 shown in said figures do not show actual dimensions.

In embodiments according to the invention, the multilayer protective structure 6 comprises a hafnia layer 61 and an alumina layer 62, also referred to as a bilayer protective structure 6. Fig. 11A shows an example of the multilayer protective structure 6, particularly the bilayer protective structure 6, where the alumina layer 62 deposited on the optical microstructure 3 and the hafnia layer 61 deposited on the alumina layer 62. In another example of the bilayer protective structure 6, the hafnia layer 61 is deposited on the optical microstructure 3 and the alumina layer 62 is deposited on the hafnia layer 61.

In embodiments of the invention, the multilayer protective structure 6 further comprises a third layer 63, also referred to as a trilayer protective structure 6, wherein the third layer 63 is deposited on the outer/top layer of the bilayer protective structure 6 disclosed herein. For example, the third layer 63 is deposited on one of the hafnia layer 61 and the alumina layer 62. It will be understood that the trilayer protective structure 6 may comprise the third layer 63 and the bilayer protective structure 6 as described herein. In embodiments of the invention, the trilayer protective structure comprises the third layer 63, wherein the bilayer protective structure 6 disclosed herein is deposited on the third layer 63. For example, one of the hafnia layer 61 and the alumina layer 62 is deposited on the third layer 63.

In preferred embodiments of the invention, the third layer 63 is a further hafnia layer 63. Alternatively, the third layer 63 is from the group of typical materials described herein, such as CRAS, SiC, DLC, TiO₂, or Al₂O₃.

In preferred embodiments of the invention, where the protective structure 6 comprises two hafnia layers 61, 63, the alumina layer 62 is considered to be "sandwiched". It will be understood by the term "sandwiched" that the alumina layer 62 is deposited between the hafnia layer 61 and the further hafnia layer 63, such that the alumina layer 62 is deposited on the further hafnia layer 63 and the hafnia layer 61 is deposited on the alumina layer 62.

Fig. 11B shows an example of the multilayer protective structure 6, particularly the trilayer protective structure 6 deposited on the optical microstructure 3, wherein the third layer 63 is deposited on the optical microstructure 3, the alumina layer is deposited on the third layer 63, and the hafnia layer 61 is deposited on the alumina layer 62. This structure of multiple protective layers has been shown to further provide improved mechanical properties (e.g., strength), without increasing the optical losses substantially. In another example, the trilayer protective structure 6 as described herein is deposited on the optical microstructure 3, such that the alumina layer 62 is deposited on the optical microstructure 3, the hafnia layer 61 is deposited on the alumina layer 62, and the third layer 63 is deposited on the hafnia layer 61.

In embodiments of the invention, the multilayer protective structure 6 further comprises a fourth layer 64, also referred to as a quadlayer protective structure, wherein the fourth layer 64 is deposited one of the hafnia layer 61 or the alumina layer 62. In alternative embodiments of the invention, the trilayer protective structure comprises the third layer 63, wherein the alumina layer 62 is deposited on the third layer 63 and the hafnia layer 61 is deposited on the alumina layer 62.

In preferred embodiments of the invention, the third layer 63 is a further hafnia layer 63. Alternatively, the third layer 63 is from the group of typical materials described herein, such as CRAS, SiC, DLC, TiO₂, or Al₂O₃.

In embodiments of the invention, the multilayer protective structure 6 further comprises a fourth layer 63, also referred to as a quadlayer protective structure 6, wherein the fourth layer 63 is deposited on the outer/top layer of the trilayer protective structure 6 disclosed herein. For example, the fourth layer 64 is deposited on one of the hafnia layer 61, the alumina layer 62 and the third layer 63. It will be understood that the quadlayer protective structure 6 may comprise the fourth layer 64 and the trilayer protective structure 6 as described herein. In embodiments of the invention, the quadlayer protective structure 6 comprises the fourth layer 64, wherein the trilayer protective structure 6 disclosed herein is deposited on the fourth layer 64.

Fig. 11C shows an example of the multilayer protective structure 6, particularly the quadlayer protective structure 6 deposited on the optical microstructure 3, wherein the fourth layer 64 is deposited on the optical microstructure 3, the third layer 63 is deposited on the fourth layer 64, the alumina layer 62 is deposited on the third layer 63, and the hafnia layer 61 is deposited on the alumina layer 62. This structure of multiple protective layers has been shown to further provide improved mechanical properties (e.g., strength), without increasing the optical losses substantially. In another example, the quadlayer protective structure 6 as described herein is deposited on the optical microstructure 3, such that the third layer 63 is deposited on the optical microstructure 3, the alumina layer 62 is deposited on the third layer 63, the hafnia layer 61 is deposited on the alumina layer 62, and the fourth layer 64 is deposited on the hafnia layer 61.

In embodiments of the invention, the fourth layer 64 is from the group of typical materials described herein, such as CRAS, SiC, DLC, TiO₂, or Al₂O₃, preferably, CRAS layer 64.

In further preferred embodiments of the invention, the third layer 63 is a further hafnia layer 63 and the fourth layer 64 is a CRAS layer 64.

In embodiments of the invention, the implantable optical sensor further comprises at least one further multilayer protective structure deposited on the multilayer protective layer 6. Each of the at least one further multilayer protective structure may be identical to the multilayer protective layer 6 as described herein.

In embodiments of the invention, there is no other layer disposed on the multilayer protective structure 6, such that the outer/top layer of the multilayer protective structure 6, particularly the hafnia layer, preferably the further hafnia layer, may be in direct and prolonged contact with a (bodily) fluid (e.g., chemical or biological substances) or a bodily tissue when implanted.

In embodiments of the invention, where the implantable optical sensor further comprises at least one further multilayer protective structure deposited on the multilayer protective layer 6, there is no other layer disposed on the at least one further multilayer protective structure.

For simplicity, we shall refer to specific combinations of layers in the multilayer protective structure, as hafnia-alumina layers (i.e., the hafnia layer 61 deposited on the alumina layer 62), as hafnia-alumina-third layers (i.e., the hafnia layer 61 deposited on the alumina layer 62 deposited on the third layer 63) or as hafnia-alumina-hafnia layers (i.e., the hafnia layer 61 deposited on the alumina layer 62 deposited on the further hafnia layer 63). It will be understood that each specific combination is read from left to right, wherein the first word on the left is deposited on the second word subsequent to (i.e. to the right of) the first word, and the second word is deposited on the third word subsequent to (i.e. to the right of) the second word, etc.

In embodiments of the invention, the multilayer protective structure 6 comprises, preferably consists of, the hafnia layer deposited on the alumina layer. The hafnia layer 61 may have a thickness of at most 20 nm, preferably within the range of 5 to 15 nm, more preferably within the range of 5 to 10 nm, even more preferably around 8 nm, and the alumina layer may have a thickness of at most 40 nm, preferably within the range of 5 to 20 nm, more preferably within the range of 10 to 20 nm, even more preferably around 15 nm. In an example, the protective structure 6 is a hafnia-alumina structure with a 10nm-20nm thickness, respectively (i.e., 10nm of hafnia layer and 20nm of alumina layer). It will be appreciated that other combinations of thicknesses will be understood by a person skilled in the art, as follows (hafnia-alumina): 5nm-10nm, 10nm-10nm, 15nm-10nm, 20nm-10nm, 5nm-15nm, 10nm-15nm, 15nm-15nm, 20nm-15nm, 5nm-20nm, 10nm-20nm, 15nm-20nm, 20nm-20nm, 5nm-25nm, 10nm-25nm, 15nm-25nm, 20nm-25nm, 5nm-30nm, 10nm-30nm, 15nm-30nm, 20nm-30nm, etc. It will be appreciated and understood from the ranges in this paragraph that the thickness of the protective structure 6 is at most 60 nm, preferably within the range of 10 nm to 35 nm, more preferably within the range of 10 nm to 30 nm, even more preferably around 23 nm, 24 nm or 25 nm. Furthermore, it will be appreciated and understood that the ranges in this paragraph also apply to embodiments of the invention where the alumina layer is deposited on the hafnia layer.

In further embodiments of the invention, the thickness of the hafnia layer 61 is less than or equal to, preferably less than or equal to 50%, of the thickness of the alumina layer 62, as shown in some of the ranges above. Further examples of combinations of thicknesses, not disclosed above but are also part of the invention, will also be understood by the skilled person, such as the following (hafnia-alumina): 5nm-10nm, 5.5nm-11nm, 6nm-12nm, 6.5nm-13nm, 7nm-14nm, 7.5nm-15nm, 8nm-16nm, 8.5nm-17nm, 9nm-18nm 9.5nm-19nm, etc.

In highly preferred embodiments of the invention, the multilayer protective structure 6 comprises, preferably consist of:
- the hafnia layer 61 having a thickness of at most 20 nm, preferably within the range of 5 to 15 nm, more preferably within the range of 5 to 10 nm, even more preferably around 8 nm, deposited on the optical microstructure 3;
- the alumina layer 62 having a thickness of at most 40 nm, preferably within the range of 5 to 30 nm, more preferably within the range of 10 to 30 nm, even more preferably around 15 nm, deposited on the hafnia layer 61; and
- the third layer 63 (e.g., the further hafnia layer 63) having a thickness of at most 20 nm, preferably within the range of 5 to 15 nm, more preferably within the range of 5 to 10 nm, deposited on the alumina layer.
In highly preferred embodiments of the invention, where the third layer is a further hafnia layer 63, the protective structure 6 is a hafnia-alumina-third structure with around 10nm-30nm-10nm thicknesses, particularly around 11nm-28nm-11nm, respectively. It will be appreciated that other combinations of thicknesses will be understood by a person skilled in the art, such as the following (hafnia-alumina-third): 5nm-10nm-5nm, 5nm-15nm-5nm, 5nm-20nm-5nm, 5nm-25nm-5nm, 5nm-30nm-5nm, 10nm-10nm-10nm, 10nm-15nm-10nm, 10nm-20nm-10nm, 10nm-25nm-10nm, etc. It will be appreciated and understood from the ranges in this paragraph that the thickness of the protective structure 6 is at most 80 nm, preferably within the range of 15 nm to 50 nm, more preferably within the range of 20 nm to 40 nm, even more preferably around 30 nm, 31 nm or 32 nm. Furthermore, It will be appreciated and understood from the ranges in this paragraph that the thickness of the protective structure 6 is at most 60 nm, preferably within the range of 10 nm to 35 nm, more preferably within the range of 10 nm to 30 nm, even more preferably around 23 nm, 24 nm or 25 nm. Furthermore, it will be appreciated and understood that the ranges in this paragraph also apply to embodiments of the invention with a different order of layers deposited on each other, as disclosed herein.

In further preferred embodiments of the invention, the thickness of the third layer 63 (e.g., the further hafnia layer 63) and/or the thickness of the hafnia layer 61 may be less than or equal to 50% of the thickness of the alumina layer 62, as shown in some of the ranges above. This ensures that the protective structure is as thin as possible without compromising the protective effect thereof, preferably the corrosion resistance. Further examples of combinations of thicknesses, not disclosed above but are also part of the invention, will also be understood by the skilled person, such as the following (hafnia-alumina-third): 5nm-10nm-5nm, 5.5nm-11nm-5.5nm, 6nm-12nm-6nm, 6.5nm-13nm-6.5nm, 7nm-14nm-7nm, 7.5nm-15nm-7.5nm, 8nm-16nm-8nm, 8.5nm-17nm-8.5nm, 9nm-18nm-9nm, 9.5nm-19nm-9.5nm, etc.

In even further preferred embodiments of the invention, the hafnia layer 61 and the third layer 63 (e.g., the further hafnia layer 63) have the same thickness, as shown in some of the ranges above corresponding to the hafnia layer 61 and the third layer 63 or the hafnia layer 61 and the further hafnia layer 63, respectively. In other embodiments of the invention, the hafnia layer 61 has a thickness less than or equal to the thickness of the third layer 63 (e.g., the further hafnia layer 63), preferably less than or equal to 50% of the thickness of the third layer 63 (e.g., the further hafnia layer 63), such that the optical microstructure 3 can be better protected, preferably such that the alumina layer 62 can also be better protected.

In embodiments of the present invention, the quadlayer protective structure 6 comprises the fourth layer 64 whose thickness is less than or equal to 50% of the combined thickness of the first, second and third layers 61-63. This ensures that the quadlayer protective structure 6 provides optimized performance (i.e., minimized optical loss) without compromising the reduced thickness of the implantable optical sensor. An example this is provided below

The protective structure 6 or each layer therein may be nonporous, e.g. the protective structure 6 comprises one or more layers having a material which is non-porous in nature. This prevents occurrence of porosity channels or pin holes in said one or more layer of the protective structure 6 which would allow a chemical or biological fluid to make a direct contact with the optical microstructure 3 during operation, thereby degrading the material and optical performance of the optical sensor 1. By having a non-porous protective structure 6 or non-porous layers therein, any direct exposure of the optical interaction area 4 with a hostile sensing environment can be prevented, even after a long term operational use.

The protective structure 6 may be optically transparent (low optical losses). In a further embodiment, it can be transparent in a visible wavelength region or an infrared (IR) region of the electromagnetic spectrum. This can be in the near IR wavelength range (between 700 nm and 2500 nm) or in the mid IR wavelength range (2.5 µm to 8 µm). The optical sensor 1 may be adapted for use in those wavelength regions where for example, glucose has particular absorption peaks or scattering resonances. For example, the optical sensor 1 may be adapted for operating in the first-overtone band (1500 nm to 1850 nm) and/or the combination band (2080 nm to 2325 nm) where glucose has numerous absorption bands and water has relatively lower absorption. The selection of which wavelength range is used could for example also be based on the available radiation sources. Additional IR wavelength ranges are described below.

### Optical Microstructure

As is understood, the optical microstructure 3 described hereon corresponds in many aspects and features with the optical microstructure 3 described with reference to Figs. 1-4.

Although most of the light in the optical microstructure 3 is confined within the guiding layer, a small portion, called the evanescent field, extends out into the external medium. The evanescent field is used to interact with the environment for e.g. trapping, sensing, exciting. This evanescent field falls off exponentially as the distance from the waveguide surface increases. E.g. in the case of a sensing application, the variation of the evanescent tail of the optical mode changes the effective refractive index of the guided mode or its amplitude, which can respectively be used as a means for sensing; called evanescent field sensing.

The optical microstructure 3 of the present invention optical assembly embodiments of the invention can be implemented as part of a Photonic Integrated Circuit (PIC), which refers to a variety of forms and material systems used for making a photonic circuit. In one embodiment of the present invention, the optical assembly 1 comprises at least one photonic integrated circuit device. In a further embodiment of the present invention, the optical microstructure 3 is based on platforms such as Silicon-on-Insulator (SOI), silicon nitride, silicon rich or stoichiometric silicon, InP, TiO₂ semiconductor membranes, polymer, glass/silica, AlxGa1-xAs, InxGa1-xAsyP1-y and plasmonic (e.g. metal nano-particles, metal layers). The optical microstructure 3 can be an integrated optical component, such as an integrated optical cavity, an integrated optical resonator, an integrated optical interferometer, an integrated optical coupler, an optical waveguide, a taper, a tuneable filter, a phase-shifter, a grating, a photonic crystal, a modulator, a detector, a source, a multiplexer, a demultiplexer or a combination thereof, embedded, integrated or patterned in the substrate 2. The optical microstructure 3 can be either active or passive. The optical microstructure 3 is integrated with the substrate as fully embedded or partially embedded structure, in order to form the optical interaction area 4 on the part of the substrate 2. The reference made to the thickness of the optical microstructure in the present description refers to the thickness of its guiding layer (e.g. the thickness of the device layer (Si) in the case of a SOI optical waveguide or the thickness of a silicon nitride optical waveguide), thus excluding the thickness of the substrate 2.

Optical grade Si and silicon nitride waveguides or a combination of both (e.g., a silicon nitride on top of SOI) which are used to transport light through optical microstructures 3 dissolve, pit and disintegrate when used in aqueous or aggressive chemical environments. In applications where the light in the optical microstructure 3 interacts with the environment e.g. a sensing activity, the deterioration of the quality of the optical microstructure 3 and the associated optical interaction area 4 has a direct impact on the performance of the optical assembly 1. The substance exposed to the optical assembly 1 may be a target analyte for a sensor, the analyte being present in for example in tissue, bodily fluid such as interstitial fluid, urine or blood, etc. Alternatively, the optical assembly 1 may be used to sense presence of a chemical fluid when referring to the operation of the optical assembly 1 in e.g. a fermentation tank, in a fuel tank or in fuel pipeline which is typically used in for example in a petrochemical environment. As discussed above, the protective structure 6 enables optical interaction of the optical microstructure 3 with the environment without changing its structural, physical, optical or chemical properties over time. Due to this reason polymer layers which are susceptible to chemical or biological degradation, or those which would affect the optical properties of the sensor are not chosen as a protective structure 6. All other areas of the device could, but must not be, protected with a polymer.

The propagation of the guided wave in the optical microstructure 3 can be either a transverse-electric (TE) polarized guided wave or a transverse-magnetic (TM) polarized guided wave. A TE wave has its electric field vector parallel to the surface of the planar waveguide and for a TM wave has its electric field vector perpendicular to the planar waveguide surface. The overall sensitivity of an optical microstructure 3 depends strongly on the waveguide material and its design. Specifically, optimizing the overlap of the exponentially decaying electric field with thickness of the protective structure 6 and maximizing the electric field strength at the waveguide surface, can optimize detection sensitivity of the optical assembly 1. In practice, a thin, protective structure 6 leads to have a high amount of evanescent field outside the optical microstructure 3 compared to a thicker protective structure 6. The protective structure 6 can be applied to any type of optical microstructure 3, irrespective of which polarized guided wave the optical microstructure 3 supports. The advantage of using a thin anti-corrosive protective structure 6 is that it allows optical interaction of the entire waveguide of the optical microstructure 3 with its environment without surface deterioration thereof, and as a result a high longevity in a biological or other corrosive environment.

In an exemplary embodiment, the optical microstructure 3 is a waveguide spiral of certain length (typically the optimum length for maximal interaction with analyte in the cladding region) exposed to the environment. The light is guided by the spiral waveguide, configured to have the evanescent tail of the guided modes or mode overlapping the environment. The interaction of the light with the environment will impact the spectral amplitude of the light.

In an exemplary embodiment, the optical microstructure 3 is a micro-ring resonator (MRR), a compact wavelength selective device. In the case of a three port MRR, three ports are provided which are known as 'In port' (coupling in light), 'Through Port' (coupling out light) and 'Drop port' (coupling out light). When an optical signal passes through the 'In port' of a MRR, a part of the optical signal is evanescently coupled into the cavity which propagates around the ring and interferes with a later arriving part of the incoming signal from the 'In port'. Destructive interference (of the cavity field and the field of the incoming signal) results in passing most of the light to the 'Through port' and constructive interference will result in most of the input power to circle the ring and eventually appear in the 'Drop port', again through evanescent coupling. In other words, light around resonant wavelengths appears as peaks in the 'Drop port' and as dips in the 'Through port'. The resonant wavelengths of the MRRs are highly affected by a change in its evanescent field which is utilised for using it as a strong evanescent field optical for biological and chemical applications. More general, depending on the loss of the system (propagation loss and coupling loss) the light stays inside and goes around the ring for 'N' number of times as determined by the quality factor of the ring. This on one hand effectively increases the interaction with the analyte and on the other hand is also extremely sensitive to any small change in the surrounding environment.

In an exemplary embodiment, the optical microstructure 3 is a guiding structure based on a slot waveguide. A slot waveguide comprises two arms of high refractive indices separated by a slot region of low refractive index. In optical slot waveguides (e.g. fabricated in SOI technology by either e-beam or deep-UV lithography) the electric field discontinuity at the interface between high index contrast materials enables high optical confinement inside a nanometer scale area (gap region) of low-index material. A variety of optical microstructures can be realized using slot waveguides such as spiral waveguides, micro-ring resonators, disk resonators and one dimensional photonic crystals. The very high field intensity build up in the slot region combined with the effective refractive index of the slot guiding structure is very sensitive to changes in the refractive index of its environment making it an efficient optical sensor.

In embodiments of the invention, the optical microstructure may be a mono-modal optical waveguide, which means that the optical microstructure is an optical waveguide suitable for mono-modal wave propagation. It is noted that the optical waveguide may be suitable for multimode wave propagation. The mono-modal optical waveguide can allow for simple and cost-effective manufacturing of the implantable optical sensor. Additionally, the mono-modal optical waveguide can allow for more effective control of waveguide cross-section with high precision and low loss. Furthermore, the combination of evanescent field sensing with the multilayer protective structure 6 provides improved reliability of the sensing of substances, while ensuring single mode evanescent sensing.

Evanescent field sensing utilises optical microstructures 3 supporting single mode and/or multimode wave propagation. Multimode optical microstructures 3 may have a thickness greater than the wavelength of the light used for absorption by the compound to be measured. However, due to relatively low levels of evanescent field intensity, detection methods are limited for sensing applications. Single mode waveguides typically comprise a very thin (less than the wavelength of the light) high dielectric index core deposited on a low index cladding. The films are typically fabricated using thin film deposition techniques or epitaxial growth. Single mode waveguides with large index differences between the core and the cladding (high contrast waveguide systems) offer greater sensitivity due to the high field intensity at the surface. Single mode planar waveguides with high index contrast enable a rapid decay of the evanescent field away from the waveguide surface with no appreciable intensity beyond one-half the wavelength of the light (~250 nm - 300 nm), while low contrast mono-mode and multi-mode configurations typically exhibit a penetration depth of 1-2 µm.

Thus, the present invention provides a balance of having sufficient evanescent tail in the substance (e.g., analyte) while supporting mono-modal wave propagation in combination with sufficient corrosion protection.

In embodiments of the invention, the optical microstructure 3 may be deposited on a silicon-on-insulator (SOI) based substrate 2. Thus, the optical microstructure 3 can allow a high level of miniaturization, which is advantageous. Furthermore, light can be efficiently coupled in and out the optical microstructure 3, e.g. by means of a grating coupler or another coupling element. Using SOI also has some technological advantages. Due to the CMOS industry, silicon technology has reached a level of maturity that outperforms any other plane chip manufacturing technique by several orders of magnitude in terms of performance, lithographical precision, reproducibility and throughput. Nano-photonic integrated circuits can be fabricated with wafer scale-processes, which means that a wafer can contain a high number of photonic integrated circuits. Combined with the commercial availability of large wafers at a relative moderate cost, this means that the price per optical sensor can be very low.

In embodiments of the invention, the optical microstructure 3 waveguide may be a silicon nitride optical waveguide, preferably the silicon nitride may be deposited on the SOI based substrate 2. This enables the combination of the passive optical functionalities implemented in the silicon nitride optical waveguide with the active functionalities in the SOI. The silicon nitride optical waveguide having a refractive index lower than Si but higher than silica (silicon dioxide or SiO₂) allows for effective evanescent field sensing. The moderate refractive index contrast of silicon nitride reduces the silicon nitride optical waveguides' vulnerability to sidewall roughness scattering. Thus, silicon nitride optical waveguides allow one to implement unique functionalities on a very compact footprint and at the same time make the waveguide less prone to scattering losses caused by nm-scale roughness of the sidewalls of the waveguide, as is typically seen with Si optical waveguides. It will also be appreciated that silicon nitride has low thermal-optic coefficient making silicon nitride optical waveguides more robust against temperature variations.

The silicon nitride waveguide may have a thickness in the range of 50 to 200 nm, preferably in the range of 100-150 nm, more preferably a thickness of around 125 nm.

The reference made to light or radiation in the present application refers to electromagnetic radiation. The light envisaged is radiation having a suitable wavelength or wavelength range for sensing, i.e. detecting or imaging, a substance. In some embodiments of the invention light used will be visible radiation or IR radiation, e.g. near IR radiation or mid IR radiation. In some embodiments of the invention, the radiation has a wavelength or wavelength range between 700 nm and 2500 nm, or between 2.5 µm and 8 µm, or a combination thereof, although the present invention embodiments of the invention are not limited thereto. For example the fabrication and integration technologies for the silicon photonics are well developed in the telecommunication wavelength range which is centred around 1550 nm, which could be exploited for easy, reliable and cost-effective manufacturing of the implantable optical sensor.

In embodiments of the invention, the at least one optical microstructure may be configured for evanescent field sensing in the near infrared, IR, wavelength range, between 700 nm and 2500 nm, or in the mid IR wavelength range, between 2.5 µm and 8 µm, preferably the at least one optical microstructure may be configured for evanescent field sensing in the IR range between 700 nm and 1100 nm or between 1.1 µm and 3.7 µm. SiO₂ optical waveguides have low absorption losses in the wavelength ranges between 1.1 µm and 3.7 µm, between 1.1 µm and 8 µm, between 2.5 µm and 8 µm or between 3.7 µm and 8 µm. However, silicon nitride optical waveguides have lower absorption losses and are better suited for the IR range below 1100 nm, preferably between 700 nm and 1100 nm. Thus, a silicon nitride optical waveguide deposited on a SOI based substrate 2 can allow for lower absorption losses throughout the whole range between 700 nm and 8000 nm, preferably in the IR ranges between 700 nm and 11000 nm, between 700 nm and 2500 nm or between 700 nm and 3700 nm.

In embodiments of the invention, the optical microstructure 3 may comprise or even consist of multilayer waveguides, preferably silicon nitride multilayer waveguides. Each of the silicon nitride multilayer waveguides may have a thickness in the range of 50 to 400 nm, preferably in the range of 100-150 nm, more preferably a thickness of around 125 nm. It will be appreciated that the thicknesses in the afore-mentioned ranges will comprise at least the following: 50 nm, 60 nm, 70 nm, 80 nm, 90 nm, 100 nm, 110 nm, 120 nm, 125 nm, 130 nm, 140 nm, 150 nm, 160 nm, 170 nm, 180 nm, 190 nm, 200 nm, 220 nm, 240 nm, 260 nm, 280 nm, 300 nm, 320 nm, 340 nm, 340 nm, 360 nm, 380 nm, 400 nm, etc.

In further embodiments of the invention, the optical microstructure 3 may be multilayer waveguides, preferably a combination of silicon nitride and SiO₂ multilayer waveguides. For instance, the optical microstructure 3 comprises or even consists of a plurality of interchanging silicon nitride layers and SiO₂ layers, such that a first silicon nitride layer is deposited on the substrate 2, preferably a SOI based substrate 2, and the SiO₂ (inter)layers are deposited in between two consecutive silicon nitride layers in the plurality of silicon nitride layers. The multilayer waveguides can allow the optical sensor 1 to further reduce propagation losses in the optical microstructure 3, particularly in the optical waveguides.

The protective structure 6 may have a thickness which is equal to or less than 50% of the optical microstructure 3 (e.g. an optical waveguide) in an even further embodiment. The thickness can be even smaller such as less than 30% or even less than 10%. For instance, the protective structure 6 has a thickness of 30 nm and the optical microstructure 3 has a thickness of 300 nm. Other combinations he protective structure 6 chosen to be equal to or less than 50% as a thicker layer could possibly affect the accessibility of the sensing media to the optical evanescent field in the first place and deteriorate the overall optical behaviour substantially in the second place. Due to the limited distance in which the evanescent field exists in an optical microstructure 3, the protective structure 6 is advantageously as thin as possible.

Each of the SiO₂ interlayers may have a thickness in the range of 50 to 200 nm, preferably in the range of 50-150 nm, more preferably a thickness of around 90 nm. It will be appreciated that the thicknesses the afore-mentioned ranges will comprise at least the following: 50 nm, 60 nm, 70 nm, 80 nm, 90 nm, 100 nm, 110 nm, 120 nm, 125 nm, 130 nm, 140 nm, 150 nm, 160 nm, 170 nm, 180 nm, 190 nm, 200 nm.

In preferred embodiments of the invention, the thickness of the silicon nitride layers is 300 nm and the thickness of the SiO₂ interlayer is 90 nm. In preferred embodiments of the invention, the thickness of the silicon nitride layers is 125 nm and the thickness of the SiO₂ interlayer is 90 nm.

In further embodiments of the invention, the number of silicon nitride layers ranges between 2 and 12, preferably 4. The number of SiO₂ interlayer may range between 1 and 11, preferably 3.

In embodiments of the invention, the optical microstructure 3 is configured to guide an optical signal from a location inside the implantable optical sensor to the optical interaction area and guide the optical signal away from the optical interaction area inside the implantable optical sensor after an interaction of said optical signal with bodily fluids in the optical interaction area 4. Thus, the optical signal can be confined within the implantable optical sensor 1. We provide below with reference to Figs. 5 and 6 examples of an optical sensor where the optical signal (i.e., light) being confined within the implantable optical sensor 1.

It will be understood that the optical sensor 1 described with reference to Figs. 5 and 6 may correspond in many aspects and/or features to the optical sensor 1 described with reference to Figs. 1-4. Thus, at least one of the optical microstructure 3, the optical interaction area 4, and the multilayer protective structure 6 described with reference to Figs. 1-4 may also be combined with the optical sensor 1 of Figs. 5 and 6.

Fig. 5 shows a schematic representation in cross sectional view of an even further embodiment of the optical sensor 1 of the present invention. In this embodiment the optical sensor 1 comprises a processing circuitry 7 attached to or integrated in the substrate 2. The optical sensor 1 may further comprise a radiation source 8 and a detector being connected to the processing circuitry 7. The optical sensor 1 may further comprise an interfacing circuitry 9 (e.g. an electronic circuitry) connected to the processing circuitry 7, the radiation source 8 and the detector. The optical sensor 1 in this embodiment is e.g. an implantable sensor, a (partially) immersed sensor, or a floating sensor). The processing circuitry 7, the radiation source 8, the detector and/or interfacing circuitry 9 (wired or wireless) in operation may be arranged for optical sensing of a substance in contact with the optical interaction area 4.

**As** is shown in Fig. 5, the protective structure 6 covers the optical interaction area 4 of the optical sensor 1. Detectors may be integrated in the optical sensor 1 to convert the optical signals into electrical signals. The detector can be a part of the processing circuitry 7 or can be arranged as a separate device. After processing the processed radiation at processing circuitry 7, an output signal may be generated via the interfacing circuitry 9. The interfacing circuitry 9 facilitates the transmission of data and power in and out of the optical sensor 1.

As is shown in Fig. 5, the radiation source 8 and interfacing circuitry 9 are electrically connected to and controlled by the processing circuitry 7, while the radiation source 8 and detector are in optical communication with the optical microstructure 3. The processing circuitry 7 is e.g. implemented as a photonic integrated circuit and is arranged for spectrally controlling radiation emitted from the radiation source 8 whose radiation is used for e.g. sensing a substance, and for processing the sensing signals (optically or electronically). The processing circuitry 7 may comprise electronics or fluidic circuitry which is monolithically, hybrid or heterogeneously integrated within the optical sensor 1. In other words, the optical sensor 1 may be arranged for capturing radiation that was directed from the radiation source 8 and that has interacted with the substance in contact with the optical interaction area 4 of the optical microstructure 3. The processing circuitry 7 may comprise a plurality of integrated components, such as optical waveguides, multiplexers, demultiplexers, couplers, splitters, filters and even tuneable elements. The processing circuitry 7 is adapted for processing the radiation in a wavelength dependent manner.

In at least some embodiments of the invention of the present invention the processing circuitry 7 may comprise optical components. The processing circuitry 7 for example act as a multiplexer or demultiplexer or part thereof, an interferometer or part thereof, an integrated optical cavity, an integrated optical resonator, an integrated optical coupler, a waveguide, a grating, or a combination thereof. The processing circuitry 7 may for example comprise a coupler for coupling and decoupling the radiation in and out of the chip. One example of a coupler may be an on-chip diffraction grating.

The couplers capturing the radiation on the chip may be optimized for capturing the optimum amount of light, using optical design techniques. The processing circuitry 7 may be an interferometer, a tuneable filter, an arrayed waveguide grating (AWG), a planar concave grating (PCG) or a combination thereof. It is an advantage of this embodiment that photonic integrated circuit based radiation processors may be used as processing circuitry 7 allowing miniaturization of the optical sensor 1. The miniaturization enables the device to be so compact that the natural flow of bodily fluids or the natural diffusion of substance, e.g. present in living creatures, enables the possibility for sensing and for continuous monitoring of the substance. It is also an advantage for e.g. that sensing is performed using optical characterization, therefore not requiring reagents or other auxiliary substances. These advantages result in a reliable and long term usable optical sensor 1, without the need for significant user interaction.

**Fig.** 6 shows a schematic representation in cross sectional view of an even further embodiment of the optical sensor 1 of the present invention. In this embodiment the optical sensor 1 comprises processing circuitry 7 attached to or integrated in the substrate 2; with a radiation source/ detector 8 being connected to the processing circuitry 7. An interfacing circuitry 9 is connected to the processing circuitry 7 and the radiation source / detector 8. In this embodiment, the protective layer 6 covers all the surfaces of the substrate 2 of the optical sensor 1.

In an alternative or additional embodiment, the implantable optical sensor 1 is an implantable sensor. The sensor can be for the sensing of a substance in the body of living creatures including the human being, but the invention is not limited thereto. The living creature may be any creature wherein the sensor can be implanted. It may for example be a plant or an animal, such as a mammal or non-mammal. It may be cold-blooded or warm-blooded. In some preferred embodiments of the invention, sensors are provided for sensing glucose, although also sensors are provided for sensing of other substances, such as urea, lactate, creatinine, triglyceride, protein, cholesterol, and ethanol. Alternatively or in addition thereto, the substance thus also may include tissue itself and sensing may be performed for imaging purposes, e.g. blood vessels, nerves, cancer tissue, cellular changes, etc. It is to be noticed that the wavelength at which sensing is performed mainly may be determined by the substance to be sensed. The selection of a different substance to be sensed, i.e. detected, imaged or monitored, thus may result in the need for using similar components as described for glucose, but having operability in another wavelength region or converters therefore. For the sake of convenience, embodiments of the invention of the present invention will be further described with reference to glucose sensing, but it will be clear to the person skilled in the art that the description in the embodiments of the invention and examples is mutates mutandis applicable to embodiments of the invention of substance sensing in a different wavelength or of a different substance. The sensor may be adapted for example by a particular detecting element and/or radiation source as well as by optical components used in the optical processor, to be used in a the near IR radiation wavelength range, e.g. in a range between 700nm and 2500nm or any other range as described herein.

The photonic and/or electronic components of the optical sensor 1 can be integrated for example monolithically, heterogeneously or by a hybrid method. Monolithic integration is the integration technology that uses a single processing flow to process the diverse components potentially using different materials, e.g. epitaxial grown germanium detectors in silicon photonics Integrated Circuit (IC). Heterogeneous integration is the integration technology for which the components are processed in separate process flows, which are then integrated at die or wafer level, e.g. BCB bonding, wafer bonding, other bonding schemes or 3D integration. Hybrid integration is the integration of components or materials on processed photonic integrated platforms, e.g. flip-chipping of detectors, bumping, gluing, wire bonding, co-packaging, etc.

In embodiments of the invention, the optical microstructure 3 is configured to guide an optical signal from a location (e.g., the radiation source 8) inside the implantable optical sensor to the optical interaction area 4 and guide the optical signal away from the optical interaction area 4 (e.g., to the radiation detector 8) inside the implantable optical sensor 1 after an interaction of said optical signal with bodily fluids in the optical interaction area 4. Thus, the optical signal can be confined within the implantable optical sensor 1, preferably within the optical microstructure 3 (e.g., the optical waveguide).

The present invention in a further aspect relates to a method for manufacturing an optical assembly, e.g. an optical assembly 1 according to any one of the embodiments of the invention described herein, the method comprising providing a substrate 2 with an integrated optical microstructure 3 forming an optical interaction area 4, and providing a protective layer 6 at least over the optical interaction area 4. As discussed above, the protective layer 6 may be provided conformal to an external surface of the optical interaction area 4, for which the processing alternatives as discussed above may be advantageously used. The deposition techniques used (especially for depositing the protective layer 6) may be selected to enhance or optimize one or more of the protective layer 6 characteristics. In an alternative embodiment, the deposition method may be optimized to have the protective layer 6 provided as a non-porous layer.

The method of applying the protective layer 6 may be using one of many thin film fabrication technologies which are known as such. For example this can be Chemical Vapour Deposition (CVD), Plasma Enhanced Chemical Vapour Deposition (PECVD), Atomic Layer Deposition (ALD), sputtering, pulsed laser deposition (PLD) or a Molecular Beam Epitaxy (MBE) deposition technique. When using a PECVD method, it is possible, but not restricted to the use of silane and methane as main gases for depositing SiC or a DLC layer as protective layer 6 on top of the optical microstructure 3 without affecting its optical transparency and further optical characteristics. The protective layer 6 can be deposited and structured using identical methods of manufacturing as are used for manufacturing the optical microstructure 3 (e.g. structured and patterned by photolithography and dry reactive etching methods).

The present invention in a further aspect relates to a use of a multilayer protective structure 6, preferably the multilayer protective structure 6 according to any of the embodiments of the invention described herein, for covering at least an optical interaction area 4 in an optical sensor 1, preferably the implantable optical sensor 1 according to any of the embodiments of the invention described herein.

The present invention has been described above with reference to a number of exemplary embodiments of the invention as shown in the drawings. Modifications and alternative implementations of some parts or elements are possible, and are included in the scope of protection as defined in the appended claims.

### Examples

Samples containing wafer pieces were coated with specific test materials as described hereinafter. The samples having 5 mm x 10 mm wafer pieces containing optical waveguides.
- Sample A (referred to as sample D13 in the Figures): Hafnia-alumina-hafnia trilayer protective structure with thickness of around 10nm-30nm-10nm, respectively, was deposited using ALD on the optical interaction area of an optical sensor (i.e., a hafnia layer deposited on the optical microstructure or optical interaction area, an alumina layer deposited on the hafnia layer, and a further hafnia layer deposited on the alumina layer);
- Sample B (referred to as sample D10 in the Figures): CRAS protective layer with thickness of around 30 nm was deposited using chemical vapor deposition (CVD) on the optical interaction area of an optical sensor;
- Sample C (referred to as sample D17 in the Figures): the layers of a hafnia-alumina-hafnia trilayer with thickness of around 10nm-30nm-10nm, respectively, were deposited using ALD on a CRAS protective layer with thickness of around 30 nm which was deposited using chemical vapor deposition (CVD) on the optical interaction area of an optical sensor (i.e., Hafnia-alumina-hafnia trilayer of sample A deposited on CRAS of sample B deposited on the optical microstructure or optical interaction area).

For each sample, three different waveguides dimensions were used each of which providing an optimized performance (i.e., minimized optical loss) around a specific wavelength range:
- Waveguide 1 (referred to as wire open 1600 in the Figures): a rectangular waveguide having a width of 0.9 µm and a thickness of around 380 nm, which provides a target optimized performance up to a wavelength of around 1600 nm;
- Waveguide 2 (referred to as wire open 2100 in the Figures): a circular waveguide having a radius of 1.6 µm and a thickness of around 380 nm, which provides a target optimized performance up to a wavelength of around 2100 nm;
- Waveguide 3 (referred to as wire open 2300 in the Figures): a circular waveguide having a radius of 1.8 µm and a thickness of around 380 nm, which provides a target optimized performance up to a wavelength of around 2300 nm.

**The** combinations of specific sample and waveguide described above will be referred to herein as "sample.waveguide" as follows: A.1, A.2, A.3, B.1, B.2, B.3, C.1, C.2 and C.3.

### Example 1

**Fig.** 7 shows a schematic representation of comparative results of an experiment performed on an implantable optical sensor according to an embodiment of the present invention. Fig. 7 shows overview transmission electron microscopy (TEM) images of a silicon photonics substrate of an optical sensor in direct and prolonged contact (for 21-22 months) with a (bodily) fluid (e.g., chemical or biological substances) or a bodily tissue.

The experiment is a comparative experiment performed on two optical sensors, the first having the CRAS according to sample B (left image) and the second having the hafnia-alumina protective trilayer according to sample A (right image).

Fig. 7 shows transmission electron microscope (TEM) images of two implantable optical sensors in a comparative experiment after 21 to 22 months of being exposed to a hostile (i.e., corrosive) environment as described above. The first implantable optical sensor having a CRAS protective layer, shown on the left, and the second implantable optical sensor having the multilayer protective structure according to the present invention, particularly the multilayer protective structure being a hafnia-alumina-hafnia structure. The optical microstructure, particularly the waveguide, in each of the two optical sensors has a thickness of around 300 nm.

In Fig. 7, the left image shows a first implantable optical sensor comprising a substrate 71 (e.g., a silicon photonics substrate as disclosed above), a first optical microstructure 72 (e.g. a waveguide as disclosed above) forming a first optical interaction area, and a CRAS protective layer 73 covering the first optical microstructure 72 at the first optical interaction area. The right image shows a second implantable optical sensor comprising a substrate 76 (e.g., a silicon photonics substrate as disclosed above), a second optical microstructure 77 (e.g. a waveguide as disclosed above) forming a second optical interaction area, and a hafnia-alumina-hafnia multilayer protective layer 78 covering the second optical microstructure 77 at the second optical interaction area.

**As** can be seen in Fig. 7, after 21 to 22 months of exposure to the hostile (i.e., corrosive) environment, the second optical microstructure 77 at the second optical interaction was better protected from corrosion than the first optical microstructure 72 at the first optical interaction area. This can be seen from the larger dimensions of the second optical microstructure 77 compared to the first optical microstructure 72. The first optical microstructure 72 is seen to have been corroded to at least 50% of the second optical microstructure 77.

The present inventors have observed that reducing the thickness of the multilayer protective structure to a total of 30 nm also provided comparable results to the those described with reference to Fig. 7.

Additionally, the present inventors have further observed that the combination of both CRAS and the hafnia-alumina-hafnia trilayer, particularly the hafnia-alumina-hafnia trilayer deposited on the CRAS layer, provided improved protection even after 21 to 22 months. This is due to the fact that the Hafnia-alumina-hafnia trilayer protects the underlying CRAS layer.

As can be observed, the multilayer protective structure provides stable and longer lasting protection of the optical microstructure.

### Example 2

Figs. 8A-8C show graphical representations of an experiment performed on sample A.

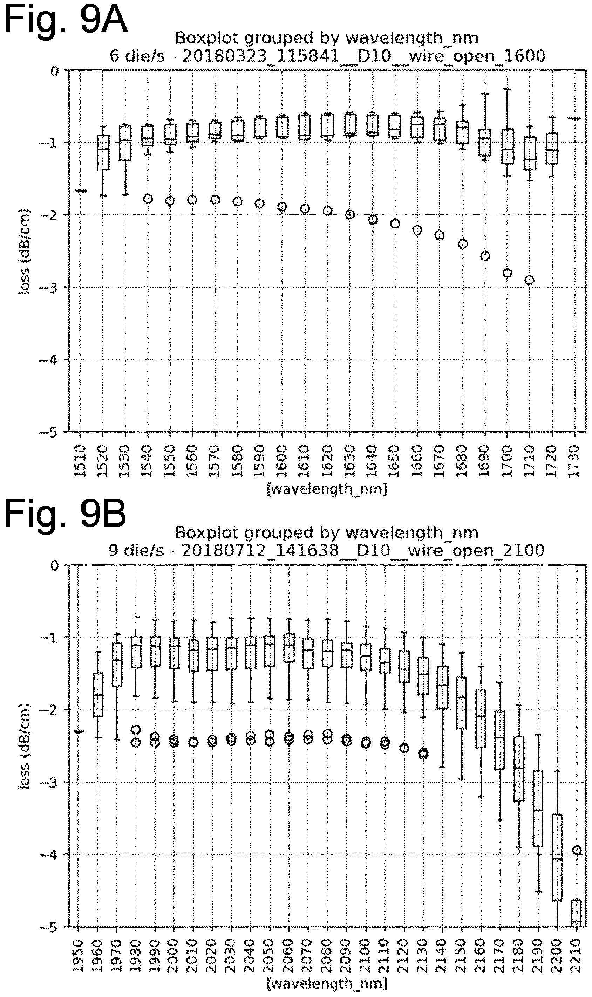

Figs. 9A-9C show graphical representations of an experiment performed on sample B.

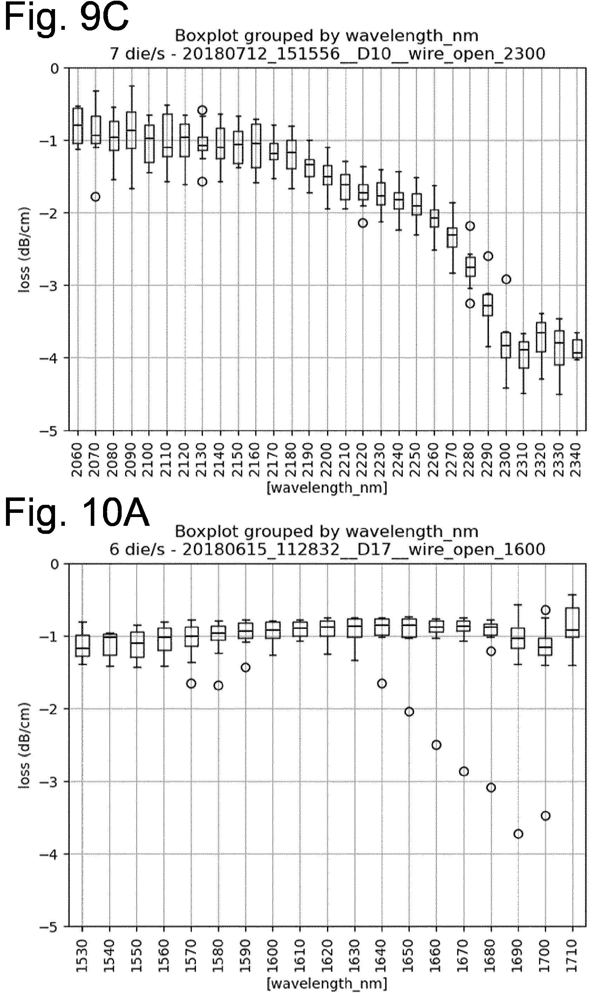

Figs. 10A-10C show graphical representations of an experiment performed on sample C.

Optical loss measurements (in dB/cm) between in specific wavelength ranges were performed on a wafer having a specific number of dies, as described hereon.

Fig. 8A shows boxplots of the optical loss measurements performed on 6 dies of the wafer of A.1 (i.e., sample A and waveguide 1) in the wavelength range of 1520-1720 nm. As can be observed, all the measurements in the wavelength range of 1520-1700 nm are lower than a loss of 1 dB/cm (i.e., higher than -1 dB/cm). In the specific wavelength of 1710 nm there is variability extending more than a loss of 1 dB/cm (i.e., lower than -1 dB/cm), however, the higher quartile still shows a lower loss than 1 dB/cm (i.e., lower quartile still higher than -1 dB/cm). In the specific wavelength of 1720 nm, the higher quartile shows a slightly higher loss than 1 dB/cm (i.e., lower quartile slightly lower than -1 dB/cm). Furthermore, the optical losses in the range of 1520-1720 nm is always lower than 1.5 dB/cm (i.e., higher than -1.5 dB/cm).

In comparison, Fig. 9A shows boxplots of the optical loss measurements performed on 6 dies of the wafer of B.1 (i.e., sample B and waveguide 1) in the wavelength range of 1520-1720 nm. As can be observed, all the measurements in the wavelength range of 1520-1720 nm show either the higher quartiles and/or outliers greater than a loss of 1 dB/cm or even 1.5 dB/cm (i.e., lower quartiles lower than -1 dB/cm or even -1.5 dB/cm).

In comparison, Fig. 10A shows boxplots of the optical loss measurements performed on 6 dies of the wafer of C.1 (i.e., sample C and waveguide 1) in the wavelength range of 1530-1720 nm. As can be observed, all the measurements in the wavelength range of 1520-1720 nm show the higher quartiles less than a loss of 1.5 dB/cm (i.e., lower quartiles lower than -1.5 dB/cm). It is understood that the outliers, which are due to the CRAS protective layer (see Fig. 9A), have reduced in number.

Therefore, the hafnia-alumina-hafnia trilayer is shown to have lower optical losses in the wavelength range of 1520-1720 nm compared to the CRAS layer.

Furthermore, by adding the hafnia-alumina-hafnia trilayer onto the CRAS protective layer to form an 80 nm multilayer protective structure, the optical performance of the sensor improved over the CRAS layer protective layer on its own in the wavelength range of 1530-1720 nm.

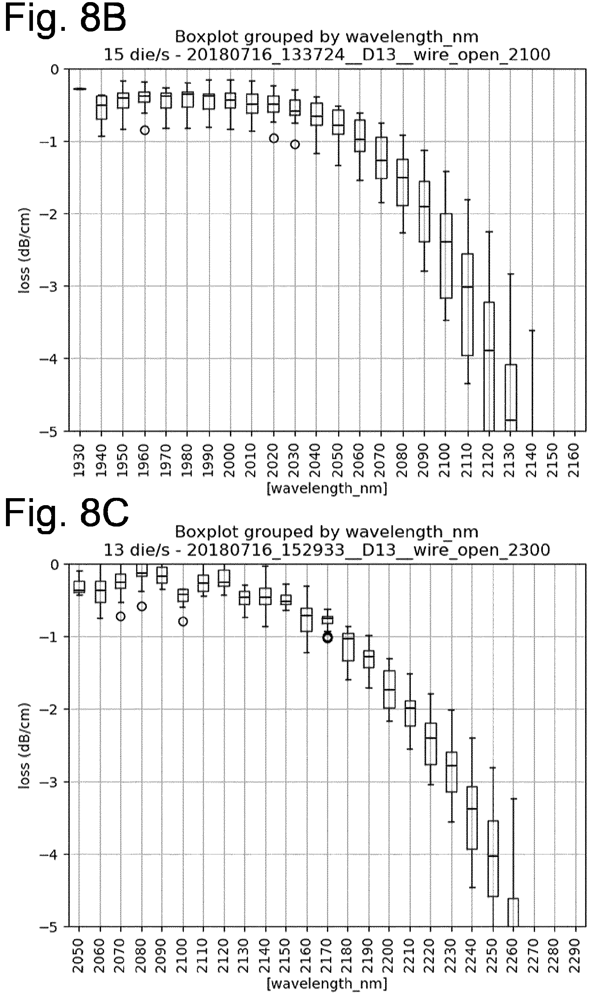

Fig. 8B shows boxplots of the optical loss measurements performed on 15 dies of the wafer of A.2 (i.e., sample A and waveguide 2) in the wavelength range of 1940-2160 nm. As can be observed, the measurements in the wavelength range of 1940-2030 nm are lower than a loss of 1 dB/cm (i.e., higher than -1 dB/cm). In the specific wavelengths of 2040 nm and 2050 nm there is variability extending more than a loss of 1 dB/cm (i.e., lower than -1 dB/cm), however, the higher quartile still shows a lower loss than 1 dB/cm (i.e., lower quartile still higher than -1 dB/cm). In the specific wavelengths of 2050 nm to 2070 nm, the higher quartile shows a slightly higher loss than 1 dB/cm (i.e., lower quartiles slightly lower than -1 dB/cm). In the specific wavelength of 2080 nm, the higher quartile shows a lower loss than 2 dB/cm (i.e., lower quartile higher than -2 dB/cm), an overall loss less than 2.5 dB/cm (i.e., higher than -2.5 dB/cm).

In comparison, Fig. 9B shows boxplots of the optical loss measurements performed on 9 dies per wafer of B.2 (i.e., sample B and waveguide 2) in the wavelength range of 1960-2210 nm. As can be observed, the measurements in the wavelength range of 1960-2090 nm show the higher quartiles being greater than a loss of 1 dB/cm (i.e., lower quartiles lower than -1 dB/cm) and outliers being greater than a loss of 2 dB/cm (i.e., lower than -2 dB/cm).

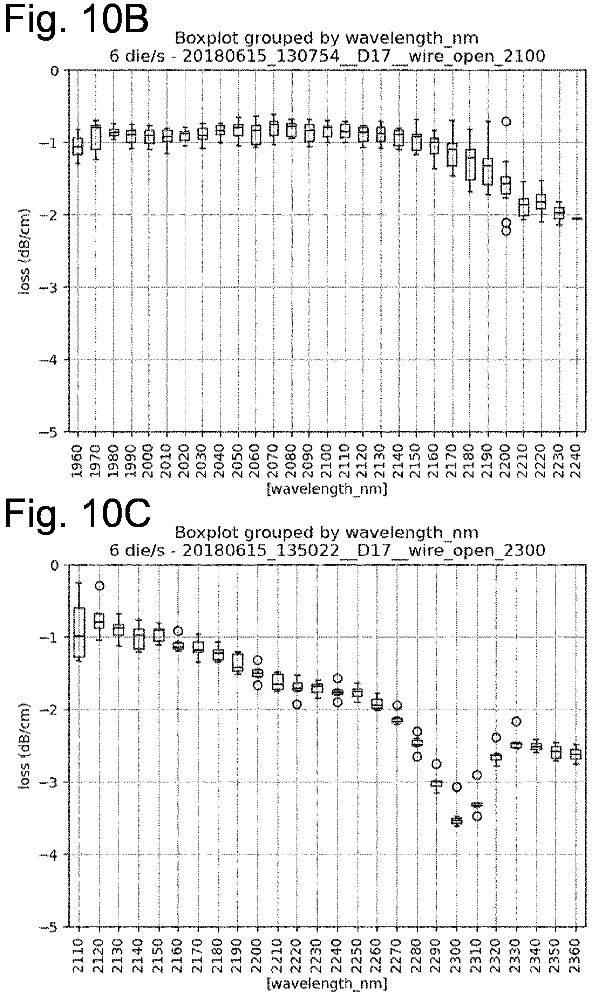

In comparison, Fig. 10B shows boxplots of the optical loss measurements performed on 6 dies of the wafer of C.2 (i.e., sample C and waveguide 2) in the wavelength range of 1960-2240 nm. As can be observed, the measurements in the wavelength range of 1960-2200 nm show the higher quartiles less than a loss of 2 dB/cm (i.e., lower quartiles lower than -2 dB/cm).

Therefore, the hafnia-alumina-hafnia trilayer is shown to have lower optical losses in the wavelength range of 1930-2080 nm compared to the CRAS layer.

Furthermore, by adding the hafnia-alumina-hafnia trilayer onto the CRAS protective layer to form an 80 nm multilayer protective structure, the optical performance of the sensor improved over the CRAS layer protective layer on its own in the wavelength range of 1960-2240 nm.

Fig. 8C shows boxplots of the optical loss measurements performed on 13 dies of the wafer of A.3 (i.e., sample A and waveguide 3) in the wavelength range of 2050-2260 nm. As can be observed, the measurements in the wavelength range of 2050-2170 nm are lower than a loss of 1 dB/cm (i.e., higher than -1 dB/cm).

In comparison, Fig. 9C shows boxplots of the optical loss measurements performed on 7 dies per wafer of B.3 (i.e., sample B and waveguide 3) in the wavelength range of 2060-2340 nm. As can be observed, the measurements in the wavelength range of 2060-2170 nm show the higher quartiles and/or outliers being greater than a loss of 1 dB/cm (i.e., lower quartiles lower than -1 dB/cm).

In comparison, Fig. 10C shows boxplots of the optical loss measurements performed on 6 dies of the wafer of C.3 (i.e., sample C and waveguide 3) in the wavelength range of 2110-2360 nm. As can be observed, the measurements in the wavelength range of 2110-2260 nm show the higher quartiles less than a loss of 2 dB/cm (i.e., lower quartiles lower than -2 dB/cm).

Therefore, the hafnia-alumina-hafnia trilayer is shown to have lower optical losses in the wavelength range of 2060-2170 nm compared to the CRAS layer.

Thus, it can be observed that the hafnia-alumina-hafnia trilayer is shown to have lower optical losses in the wavelength range of 1520-1720 nm and 1960-2170 nm compared to the CRAS layer.

Furthermore, it can be observed that by adding the hafnia-alumina-hafnia trilayer onto the CRAS protective layer to form an 80 nm multilayer protective structure, the optical performance of the sensor improved over the CRAS layer protective layer on its own in the wavelength ranges of 1530-1710 nm, 1960-2360 nm.

The inventors have observed that by increasing the thickness of the CRAS protective layer of Sample B to 50 nm, although its protective properties would improve, the optical losses would increase drastically, thereby reducing the optical performance of the optical sensor and making the sensor ineffective (e.g., reduced detection sensitivity).

As can be observed from the optical loss measurements, the multilayer protective structure of the present invention provides an unexpectedly improved optical performance (i.e., reduced optical losses) compared with other protective layers, such as CRAS.

## Claims

1. An implantable optical sensor (1) comprising:
at least one optical microstructure (3) configured for evanescent field sensing, the at least one optical microstructure (3) being positioned to form an optical interaction area (4) on a part of a surface (5) of the implantable optical sensor (1),
wherein the implantable optical sensor further comprises a multilayer protective structure (6) covering at least the optical interaction area (4), the optical sensor being **characterized in that** the multilayer protective structure (6) comprises a first layer (61) deposited on a second layer (62), the first layer (61) being a hafnia layer (61) and the second layer (62) being an alumina layer (62).

2. The implantable optical sensor according to claim 1, wherein the multilayer protective structure (6) comprises a third layer (63) deposited on the optical microstructure (3), the alumina layer (62) deposited on the third layer (63), and the hafnia layer (61) deposited on the alumina layer (62).

3. The implantable optical sensor according to claim 1, wherein the multilayer protective structure (6) comprises a fourth layer (64) deposited on the optical microstructure (3), a third layer (63) deposited on the fourth layer (64), the alumina layer (62) deposited on the third layer (63), and the hafnia layer (61) deposited on the alumina layer (62).

4. The implantable optical sensor according to claim 2 or claim 3, wherein the third layer (63) is a further hafnia layer (63) and/or wherein the fourth layer (64) is preferably a carbon-rich amorphous silicon layer (64).

5. The implantable optical sensor according to any of claims 1-4, wherein the implantable optical sensor further comprises at least one further multilayer protective structure deposited on the multilayer protective layer (6).

6. The implantable optical sensor according to any of claims 1-5, wherein the optical microstructure (3) is a mono-modal optical waveguide.

7. The implantable optical sensor according to any of claims 1-6, wherein the optical microstructure (3) is deposited on a silicon-on-insulator based substrate (2).

8. The implantable optical sensor according to any of claims 1-7, wherein the optical microstructure (3) is a silicon nitride optical waveguide.

9. The implantable optical sensor according to any of claims 1-8, wherein the hafnia layer (61) and/or the third layer (63) has a thickness of less than 50% of the thickness of the alumina layer (62).

10. The implantable optical sensor according to any of claims 3-9, wherein the fourth layer (64) has a thickness of less than 50% of the combined thickness of the hafnia layer (61), the alumina layer (62), and the third layer (63).

11. The implantable optical sensor according to claim 9 or claim 10, wherein the hafnia layer (61) and/or the third layer (63) has a thickness of at most 20 nm and the alumina layer (62) has a thickness of at most 40 nm.

12. The implantable optical sensor according to any of claims 1-11, wherein the multilayer protective structure (6) has an optical loss less than 3 dB/cm, preferably less than 2 dB/cm.

13. The implantable optical sensor according to any of claims 1-12, wherein no other layer is deposited on the multilayer protective structure (6) or the at least one further multilayer protective structure.

14. A method of manufacturing an implantable optical sensor according to any of claims 1-13, the method comprising:
providing a substrate (2); and
providing at least one optical microstructure (3) for evanescent field sensing, the at least one optical microstructure (3) being positioned to form an optical interaction area (4) on a part of a surface (5) of the substrate (2),
wherein the method further comprises providing a multilayer protective structure (6) covering at least the optical interaction area (4), **characterized in that** the multilayer protective structure (6) comprises a first layer (61) deposited on a second layer (62), the first layer (61) being a hafnia layer (61) and the second layer (62) being an alumina layer (62).

15. Use of a multilayer protective structure (6) for protecting an implantable optical sensor (1) preferably according to any of claims 1-12,
the multilayer protective structure (6) covering at least an optical interaction area (4) in the implantable optical sensor (1), **characterized in that** the multilayer protective structure (6) comprises a first layer (61) deposited on a second layer (62), the first layer (61) being a hafnia layer (61) and the second layer (62) being an alumina layer (62).

## Patentansprüche

1. Implantierbarer optischer Sensor (1), umfassend:
mindestens eine optische Mikrostruktur (3), die zur Erfassung eines abklingenden Feldes konfiguriert ist, wobei die mindestens eine optische Mikrostruktur (3) positioniert ist, um einen optischen Interaktionsbereich (4) auf einem Teil einer Oberfläche (5) des implantierbaren optischen Sensors (1) auszubilden,
wobei der implantierbare optische Sensor ferner eine mehrschichtige Schutzstruktur (6) umfasst, die mindestens den optischen Interaktionsbereich (4) bedeckt, wobei der optische Sensor **dadurch gekennzeichnet ist, dass** die mehrschichtige Schutzstruktur (6) eine erste Schicht (61) umfasst, die auf einer zweiten Schicht (62) abgeschieden ist, wobei die erste Schicht (61) eine Hafniumoxidschicht (61) ist und die zweite Schicht (62) eine Aluminiumoxidschicht (62) ist.

2. Implantierbarer optischer Sensor nach Anspruch 1, wobei die mehrschichtige Schutzstruktur (6) eine dritte Schicht (63) umfasst, die auf der optischen Mikrostruktur (3) abgeschieden ist, wobei die Aluminiumoxidschicht (62) auf der dritten Schicht (63) abgeschieden ist und wobei die Hafniumoxidschicht (61) auf der Aluminiumoxidschicht (62) abgeschieden ist.

3. Implantierbarer optischer Sensor nach Anspruch 1, wobei die mehrschichtige Schutzstruktur (6) eine vierte Schicht (64), die auf der optischen Mikrostruktur (3) abgeschieden ist, eine dritte Schicht (63), die auf der vierten Schicht (64) abgeschieden ist, die Aluminiumoxidschicht (62), die auf der dritten Schicht (63) abgeschieden ist, und die Hafniumoxidschicht (61) auf der Aluminiumoxidschicht (62) abgeschieden ist, umfasst.

4. Implantierbarer optischer Sensor nach Anspruch 2 oder Anspruch 3, wobei die dritte Schicht (63) eine weitere Hafniumoxidschicht (63) ist und/oder wobei die vierte Schicht (64) vorzugsweise eine kohlenstoffreiche amorphe Siliciumschicht (64) ist.

5. Implantierbarer optischer Sensor nach einem der Ansprüche 1-4, wobei der implantierbare optische Sensor ferner mindestens eine weitere mehrschichtige Schutzstruktur umfasst, die auf der mehrschichtigen Schutzschicht (6) abgeschieden ist.

6. Implantierbarer optischer Sensor nach einem der Ansprüche 1-5, wobei die optische Mikrostruktur (3) ein monomodaler optischer Wellenleiter ist.

7. Implantierbarer optischer Sensor nach einem der Ansprüche 1-6, wobei die optische Mikrostruktur (3) auf einem Substrat (2) auf Silicium-auf-Isolator-Basis abgeschieden ist.

8. Implantierbarer optischer Sensor nach einem der Ansprüche 1-7, wobei die optische Mikrostruktur (3) ein optischer Siliciumnitrid-Wellenleiter ist.

9. Implantierbarer optischer Sensor nach einem der Ansprüche 1-8, wobei die Hafniumoxidschicht (61) und/oder die dritte Schicht (63) eine Dicke von weniger als 50 % der Dicke der Aluminiumoxidschicht (62) aufweist.

10. Implantierbarer optischer Sensor nach einem der Ansprüche 3-9, wobei die vierte Schicht (64) eine Dicke von weniger als 50 % der kombinierten Dicke der Hafniumoxidschicht (61), der Aluminiumoxidschicht (62) und der dritten Schicht (63) aufweist.

11. Implantierbarer optischer Sensor nach Anspruch 9 oder Anspruch 10, wobei die Hafniumoxidschicht (61) und/oder die dritte Schicht (63) eine Dicke von höchstens 20 nm aufweist und die Aluminiumoxidschicht (62) eine Dicke von höchstens 40 nm aufweist.

12. Implantierbarer optischer Sensor nach einem der Ansprüche 1-11, wobei die mehrschichtige Schutzstruktur (6) einen optischen Verlust von weniger als 3 dB/cm, bevorzugt weniger als 2 dB/cm aufweist.

13. Implantierbarer optischer Sensor nach einem der Ansprüche 1-12, wobei keine andere Schicht auf der mehrschichtigen Schutzstruktur (6) oder der mindesten einen weiteren mehrschichtigen Schutzstruktur abgeschieden ist.

14. Verfahren zur Herstellung eines implantierbaren optischen Sensors nach einem der Ansprüche 1-13, wobei das Verfahren Folgendes umfasst:
Bereitstellen eines Substrats (2); und
Bereitstellen mindestens einer optischen Mikrostruktur (3) zur Erfassung eines abklingenden Feldes, wobei die mindestens eine optische Mikrostruktur (3) positioniert ist, um einen optischen Interaktionsbereich (4) auf einem Teil einer Oberfläche (5) des Substrats (2) auszubilden,
wobei das Verfahren ferner Bereitstellen einer mehrschichtigen Schutzstruktur (6) umfasst, die mindestens den optischen Interaktionsbereich (4) bedeckt, **dadurch gekennzeichnet, dass**
die mehrschichtige Schutzstruktur (6) eine erste Schicht (61) umfasst, die auf einer zweiten Schicht (62) abgeschieden ist, wobei die erste Schicht (61) eine Hafniumoxidschicht (61) ist und die zweite Schicht (62) eine Aluminiumoxidschicht (62) ist.

15. Verwendung einer mehrschichtigen Schutzstruktur (6) zum Schützen eines implantierbaren optischen Sensors (1), bevorzugt nach einem der Ansprüche 1-12,
wobei die mehrschichtige Schutzstruktur (6) mindestens einen optischen Interaktionsbereich (4) in dem implantierbaren optischen Sensor (1) bedeckt, **dadurch gekennzeichnet, dass**
die mehrschichtige Schutzstruktur (6) eine erste Schicht (61) umfasst, die auf einer zweiten Schicht (62) abgeschieden ist, wobei die erste Schicht (61) eine Hafniumoxidschicht (61) ist und die zweite Schicht (62) eine Aluminiumoxidschicht (62) ist.

## Revendications

1. Capteur optique implantable (1) comprenant :
au moins une microstructure optique (3) conçue pour la détection de champ évanescent, ladite au moins une microstructure optique (3) étant positionnée pour former une zone d'interaction optique (4) sur une partie d'une surface (5) du capteur optique implantable (1),
ledit capteur optique implantable comprenant en outre une structure de protection multicouche (6) recouvrant au moins la zone d'interaction optique (4), le capteur optique étant **caractérisé en ce que** la structure de protection multicouche (6) comprend une première couche (61) déposée sur une seconde couche (62), la première couche (61) étant une couche de hafnie (61) et la seconde couche (62) étant une couche d'alumine (62).

2. Capteur optique implantable selon la revendication 1, ladite structure de protection multicouche (6) comprenant une troisième couche (63) déposée sur la microstructure optique (3), la couche d'alumine (62) déposée sur la troisième couche (63), et la couche de hafnie (61) déposée sur la couche d'alumine (62).

3. Capteur optique implantable selon la revendication 1, ladite structure de protection multicouche (6) comprenant une quatrième couche (64) déposée sur la microstructure optique (3), une troisième couche (63) déposée sur la quatrième couche (64), la couche d'alumine (62) déposée sur la troisième couche (63), et la couche de hafnie (61) déposée sur la couche d'alumine (62).

4. Capteur optique implantable selon la revendication 2 ou la revendication 3, ladite troisième couche (63) étant une couche de hafnie (63) supplémentaire et/ou ladite quatrième couche (64) étant de préférence une couche de silicium amorphe (64) riche en carbone.

5. Capteur optique implantable selon l'une quelconque des revendications 1 à 4, ledit capteur optique implantable comprenant en outre au moins une structure protectrice multicouche supplémentaire déposée sur la couche protectrice multicouche (6).

6. Capteur optique implantable selon l'une quelconque des revendications 1 à 5, ladite microstructure optique (3) étant un guide d'onde optique monomodal.

7. Capteur optique implantable selon l'une quelconque des revendications 1 à 6, ladite microstructure optique (3) étant déposée sur un substrat à base de silicium sur isolant (2).

8. Capteur optique implantable selon l'une quelconque des revendications 1 à 7, ladite microstructure optique (3) étant un guide d'onde optique en nitrure de silicium.

9. Capteur optique implantable selon l'une quelconque des revendications 1 à 8, ladite couche de hafnie (61) et/ou ladite troisième couche (63) possédant une épaisseur inférieure à 50 % de l'épaisseur de la couche d'alumine (62).

10. Capteur optique implantable selon l'une quelconque des revendications 3 à 9, ladite quatrième couche (64) possédant une épaisseur inférieure à 50 % de l'épaisseur combinée de la couche de hafnie (61), de la couche d'alumine (62) et de la troisième couche (63).

11. Capteur optique implantable selon la revendication 9 ou la revendication 10, ladite couche de hafnie (61) et/ou ladite troisième couche (63) possédant une épaisseur inférieure ou égale à 20 nm et ladite couche d'alumine (62) possédant une épaisseur inférieure ou égale à 40 nm.

12. Capteur optique implantable selon l'une quelconque des revendications 1 à 11, ladite structure protectrice multicouche (6) présentant une perte optique inférieure à 3 dB/cm, de préférence inférieure à 2 dB/cm.

13. Capteur optique implantable selon l'une quelconque des revendications 1 à 12, aucune autre couche n'étant déposée sur la structure protectrice multicouche (6) ou ladite au moins une structure protectrice multicouche supplémentaire.

14. Procédé de fabrication d'un capteur optique implantable selon l'une quelconque des revendications 1 à 13, le procédé comprenant :
la fourniture d'un substrat (2) ; et
la fourniture d'au moins une microstructure optique (3) pour la détection de champ évanescent, ladite au moins une microstructure optique (3) étant positionnée pour former une zone d'interaction optique (4) sur une partie d'une surface (5) du substrat (2),
ledit procédé comprenant en outre la fourniture d'une structure de protection multicouche (6) recouvrant au moins la zone d'interaction optique (4), **caractérisé en ce que** la structure de protection multicouche (6) comprend une première couche (61) déposée sur une seconde couche (62), la première couche (61) étant une couche de hafnie (61) et la seconde couche (62) étant une couche d'alumine (62).

15. Utilisation d'une structure de protection multicouche (6) pour la protection d'un capteur optique implantable (1) de préférence selon l'une quelconque des revendications 1-12,
la structure de protection multicouche (6) recouvrant au moins une zone d'interaction optique (4) dans le capteur optique implantable (1), **caractérisée en ce que** la structure de protection multicouche (6) comprend une première couche (61) déposée sur une seconde couche (62), la première couche (61) étant une couche de hafnie (61) et la seconde couche (62) étant une couche d'alumine (62).
